Europäisches Patentamt

European Patent Office

Office européen des brevets

(11) Publication number: **0 328 248**
**A2**

(12) # EUROPEAN PATENT APPLICATION

(21) Application number: **89300278.2**

(22) Date of filing: **12.01.89**

(51) Int. Cl.⁴: **C12P 21/00 , C12N 5/00 , C07K 3/18 , C07K 13/00 , A61K 37/38 , A61K 39/395**

The microorganism(s) has (have) been deposited with European Collection of Animal Cell Cultures under number(s) BFR 87-70, BFR 87-124.

The title of the invention has been amended (Guidelines for Examination in the EPO, A-III, 7.3).

(30) Priority: **12.01.88 AU 6257/88**

(43) Date of publication of application:
**16.08.89 Bulletin 89/33**

(84) Designated Contracting States:
**AT BE CH DE ES FR GB GR IT LI LU NL SE**

(71) Applicant: **Bunge (Australia) Proprietary Limited**
**6th Floor 616 St. Kilda Road**
**Melbourne Victoria 3004(AU)**

(72) Inventor: **Fiddes, Rodney John,**
**Unit 2, 39 Myrnong Crescent,**
**Ascot Vale 3032, Victoria,(AU)**

(74) Representative: **Harding, Richard Patrick et al**
**Arthur R. Davies & Co. 27 Imperial Square**
**Cheltenham GL50 1RQ(GB)**

(54) **Monoclonal antibodies against a follicle-stimulating hormone.**

(57) In order to produce a monoclonal antibody against follicle stimulating hormone (FSH) a B cell capable of producing antibodies against FSH is fused with a myeloma cell. The resulting hybridoma is propagated, and an antibody produced by the hybridoma harvested. The antibody-producing hybridoma is detected utilising a radioimmunoassay together with immunohistology, and the fused cell hybrid selected is subsequently cloned into individual antibody-producing cell lines. In order to purify FSH impure FSH is passed through the monoclonal antibody against FSH bound to a matrix and the captured FSH is eluted from the matrix.

EP 0 328 248 A2

## Monoclonal Antibodies

The present invention relates to monoclonal antibodies, a method for the preparation thereof and use thereof.

Follicle-stimulating hormone (FSH) or follitropin, is a glycoprotein hormone secreted by the pituitary gland. FSH, and other pituitary glycoprotein hormones (Luteinizing hormone LH, thyroid stimulating hormone TSH,), consist of an $\alpha$ and $\beta$ subunit. The $\alpha$-subunit of these hormones is common within a species whilst the $\beta$-subunit differs and confers the specificity of each hormone.

FSH has a role in sexual function in both male and female animals. In males, it seems the most important function of FSH is in the maturation and nutrition of sperm, whilst in females FSH has a role in development of the ovum and ovulation. Exogenous FSH can be used for superovulation in embryo transfer programs, or for ovulation increases to give greater fecundity. However variation in the response of treated animals can be a major problem and may be attributed to batch variation and continents present in FSH preparations, particularly the presence of another pituitary glycoprotein hormone, LH (Luteinizing hormone)..

The major methods known in the prior art of ovine FSH (oFSH) extraction include

(i) for the production of crude extracts of oFSH from lamb pituitary glands by the use of ethanol and acid precipitation (Sherwood, et al (1970) J. Biol Chem. 245: 2329-2336), and

(ii) ammonium sulphate precipitation (Reichert, L.E. in Methods in Enzymology Vol. XXXVII Ed. O'Malley B.W. and Hardman J.G. Academic Press Inc. N.Y. 1975).

Depending on the method used unwanted proteins are precipitated out by the stepwise addition of cold ethanol or ammonium sulphate. A final addition of ethanol or ammonium sulphate precipitates the active component as well as some contaminants. These methods produce crude FSH extracts containing 0.5 - 1.0 iu/mg of crude protein.

Further purification steps using the crude pituitary extracts include gel filtration and ion-exchange chromatography (Sherwood, et al, 1970, Sairam 1979, Grimecti 1974. The purified products obtained range in potency from 110 to 188 units of oFSH per mg of total protein.

However these procedures incur large expense, are time consuming and produce only small amounts from each pituitary gland, and as a result large quantities of FSH are rarely available for veterinary use, especially FSH of a consistent potency.

Accordingly it is an object of the present invention to overcome, or at least alleviate, one or more of the difficulties related to the prior art.

Accordingly in a first aspect, the present invention provides a process for producing a monoclonal antibody against follicle stimulating hormone (FSH), which process includes
providing a B cell capable of producing antibodies against FSH, and a myeloma cell,
fusing the B cell with the myeloma cell, and
propagating a hybridoma formed thereby.

The process for producing a monoclonal antibody according to this aspect of the present invention may further include
harvesting an antibody produced by said hybridoma.

The B cell capable of producing antibodies against FSH may be selected from spleen cells and lymph node cells of a test animal. The test animal may be a mouse, rat or the like. The B cells may be obtained from an animal immunised with FSH or an immunogenic fragment thereof. A BALB/c mouse is the preferred animal for immunisation. Once-primed or hyper-immunised animals may be used as the source of antibody producing lymphocytes or splenocytes. In a particularly preferred form, hyperimmunised mouse spleen cells are used to make the fused cell hybrids.

The myeloma cell may be of any suitable type. Specialized myeloma cell lines which have been developed from lymphocyte tumours for use in hybridoma-producing fusion procedures are known in the prior art.

Several myeloma cell lines may be used for the production of fused cell hybrids, including P3/X63-Ag 8, NS-I type myeloma cells, e.g. P3/NSI/1-Ag 4-1, Sp2/0-Ag14 and S194/5.XXO.BU.1. In the example of the present invention, a NS-1-type myeloma cell (derived from BALB/c mice) is the preferred cell line.

The fusion step according to this aspect of the present invention for generating hybrids of antibody-producing spleen or lymph node cells and myeloma cells includes mixing somatic cells with myeloma cells. The proportion may vary from approximately 20:1 to 1:1, preferably 10:1, respectively. The fusion step may be carried out in the presence of an agent or agents that promote the fusion of cell membranes. It is preferred that the same species of animal serve as the source of the somatic and myeloma cells used in

2

the fusion procedure. The fusion-promoting agents used may include Sendai virus or polyethylene glycol (PEG).

Because fusion procedures produce viable hybrids at very low frequency (e.g., when spleens are used as a source of somatic cells, only one hybrid is obtained for roughly every $2 \times 10^5$ spleen cells), it is preferred to have a means of selecting the fused cell hybrids from the remaining unfused cells, particularly the unfused myeloma cells. A means of detecting the desired antibody-producing hybridomas among other resulting fused cell hybrids is also necessary.

Generally, the selection of fused cell hybrids is accomplished by culturing the cells in media that support the growth of hybridomas but prevent the growth of the myeloma cells which normally would go on dividing indefinitely. These cells are selected against in hypoxanthine/aminopterin/thymidine (HAT) medium, a medium in which the fused cell hybrids survive due to the HPRT-positive genotype of the spleen cells. The use of myeloma cells with different genetic deficiencies (e.g., other enzyme deficiencies, drug sensitivities, etc.) that can be selected against in media supporting the growth of genotypically competent hybrids is also possible.

The use of highly discriminatory screening assays during the cloning and selection of hybridoma cell lines assists in selecting for monoclonal antibodies having the desired specificity.

The detection of antibody-producing hybrids can be achieved by any one of several standard assay methods, including enzyme-linked immunoassay and radioimmunoassay techniques. Immunohistology can also be used for antibody detection and selection. The detection method preferred is a double-antibody radioimmunoassay employing radioactive-iodine labelled oFSH. Other assay techniques which have been tested may lead to false positive results.

Once the desired fused cell hybrids have been selected and cloned into individual antibody-producing cell lines, each cell line may be propagated in either of two standard ways. A sample of the hybridoma can be injected into a histocompatible animal of the type that was used to provide the somatic and myeloma cells for the original fusion. The injected animal develops tumours secreting the specific monoclonal antibody produced by the fused cell hybrid. The body fluids of the animal, such as serum or ascites fluid, can be tapped to provide monoclonal antibodies in high concentration. Alternatively, the individual cell lines may be propagated in vitro in laboratory culture vessels; the culture medium, also containing high concentrations of a single specific monoclonal antibody, can be harvested by decantation, filtration or centrifugation.

Accordingly, in a further aspect of the present invention there is provided a continuous cell line which produces a monoclonal antibody against follicle stimulating hormone including a hybridoma formed by fusing a B cell capable of producing antibodies against follicle stimulating hormone with a myeloma cell.

Preferably, the hybridoma cell line is formed by fusing an NS-1 myeloma cell with a spleen B cell obtained from a BALB/c mouse immunised with follicle stimulating hormone. An ovine follicle stimulating hormone (oFSH) preparation may be used to immunise the mouse.

The mouse is immunised with FSH extracted from lamb or pig pituitary glands combined with an adjuvant. The hybridoma cell line grows in tissue culture using a nutrient solution. The cell line when analysed for purity after repeated tissue culture passage by dilution cloning will exhibit antibody producing FSH specific clones at a frequency of 100%.

Hybridomas of interest may be dilution cloned to ensure the selection of a single cell line. Hybridomas may be dilution cloned at least four times. Particular examples of hybridoma cell lines are those designated BFR87-70 and BFR87-124 which are raised against ovine FSH.

Samples of the above-mentioned are maintained in the Bunge (Australia) Pty. Ltd. Cell Collection at 89 Flemington Road, North Melbourne, Australia. The invention described herein is not limited in scope by the hybridoma cell lines exemplified since these embodiments are intended as illustrations of one aspect of the invention and any equivalent cell lines which produce a functionally equivalent monoclonal antibody are within the scope of the invention. Samples of the following hybridoma cell lines have been deposited with the European Collection of Animal Cell Cultures (ECACC)

| Culture | Deposit No. |
|---|---|
| BFR87-70 | 89010301 |
| BFR87-124 | 89010302 |

In a still further aspect of the present invention there is provided a monoclonal antibody against follicle stimulating hormone produced from a continuous cell line which produces a monoclonal antibody against follicle stimulating hormone including a hybridoma formed by fusing a B cell capable of producing

3

antibodies against follicle stimulating hormone with a myeloma cell.

The monoclonal antibody may be produced from a hybridoma cell line as described above. Specific examples of monoclonal antibodies include those formed by hybridoma cell lines BFR87-70 and BFR 87-124 which are raised against ovine FSH, samples of which are maintained in the Cell Collection facility of Bunge (Australia) Pty. Ltd. at 89 Flemington Road, North Melbourne, Australia.

In a further aspect of the present invention there is provided a method of purifying follicle stimulating hormone (FSH) which method includes

providing a source of impure FSH, and

a monoclonal antibody against FSH bound to a matrix; and

passing the impure FSH through the monoclonal antibody matrix to capture FSH.

The method of purification may further include eluting the captured FSH from the monoclonal antibody matrix.

The monoclonal antibodies bound to the matrix may be any of the monoclonal antibodies described above.

A simple method for the purification of follicle stimulating hormone is of importance since those methods known in the prior art are both costly and time consuming, and, result in a product of low purity. A purified FSH is of importance as it provides a greatly improved biological response in-vivo. For example, cintamination with LH decreases the effects of administered FSH. High purity FSH is extremely desirable for application in human medicine.

The development of technology enabling the purification of FSH through monoclonal antibody affinity chromatography has provided a process whereby the supply of commercial quantities of a highly purified FSH product is possible.

Highly purified FSH has some distinct advantages over the impure FSH products currently available. Batch variation of commercial FSH is extensive and the quality of the in-vivo response is poor by comparison. FSH purified from impure batches of FSH using the presently described technology has little if any batch variation and provides a greatly improved response in-vivo. The improved response is evident in all species tested.

Accordingly, in a still further aspect of the present invention there is provided a purified follicle stimulating hormone (FSH). Preferably the purified FSH is a purified ovine FSH.

The matrix used may be of any suitable type. A chromatographic gel is preferred. An affinity chromatographic gel may be used. The gel sold under the trade designation AFFIGEL 10 and available from Bio-Rad Laboratories has been found to be suitable. AFFIPREP 10 and 15 (Bio-rad Laboratories) are also suitable.

The step of passing the impure FSH through the monoclonal antibody matrix may be undertaken in any suitable container. An affinity column may be used for relatively small scale work. For large scale work, a large container or vat may be used. The large container or vat may be rotated, agitated or stirred to ensure complete contact between the impure FSH and the monoclonal antibody matrix. A rotor bottle may be used as a suitable container.

The captured FSH maybe eluted from the monoclonal antibody matrix utilising any suitable elution solution. An organic acid solution may be used. A pH buffered solution may be used. A citrate/citric acid buffer may be used to remove unbound material from the column.

Stepwise elutions with 0.1M citrate/citric acid solutions with a pH of approximately 6.75 to 2.0 have been found to be suitable.

The in-vivo response to FSH may be improved through the application of the following new technologies

(i) High purity FSH, and

(ii) Monoclonal antibodies to FSH.

In a further aspect of the present invention there is provided a method of inducing oestrus and/or increasing fecundity in female animals which method includes administering to the animal an effective amount of a purified follicle stimulating hormone. Anoestrus animals are thereby induced to cycle and ovulate.

The term animals as used herein in the description and claims includes domestic animals including sheep, goats, cattle, pigs and the like as well as other animals including humans.

For sheep and goats the ovine follicle stimulating hormone may be administered in amounts of from approximately 2 to 10 iu. For cattle the follicle stimulating hormone may be administered in amounts of from approximately 2 to 20 iu.

In a further preferred aspect of the present invention there is provided a method of substantially increasing the number of ova produced during a single oestrus cycle in a female animal (superovulation). This method includes administering to the animal an effective amount of a purified follicle stimulating hormone. The number of ova produced may be increased to a level of at least about 5 preferably 15-20.

For sheep and goats the follicle stimulating hormone may be administered in amounts of from approximately 4 to 20 iu.

For cattle the follicle stimulating hormone may be administered in amounts of from approximately 4 to 20 iu.

It has now been discovered that monoclonal antibodies to follicle stimulating hormone may block and/or enhance the biological activity of follicle stimulating hormone in animals.

Control of FSH bioactivity in vivo using monoclonal antibodies is advangeous when using FSH for

    (i) superovulation,
    (ii) increasing fecundity, and
    (iii) manipulation of the oestrus cycle.

Accordingly, the methods described above may further include administering simultaneously with, or after, FSH administration to the animal approximately 0.25 to 3.0 mg of an purified monoclonal antibody against FSH formed by a hybridoma cell line.

The advantages already described for superovulation are also applied to the low dosage usage of FSH for increasing fecundity, i.e. twinning and manipulation of the oestrus cycles. By using these monoclonal antibodies with low doses of FSH the incidence of twin births in a variety of species can be better controlled. Twinning is therefor easier and more repeatable, and the numbers of unwanted triple and high multiple births are reduced.

Accordingly in a further aspect of the present invention there is provided a method of increasing ovulation to provide twinning in female animals which method includes administering to the animal an effective amount of a purified follicle stimulating hormone.

Preferably the animal to be treated is a sheep, goat or cow and the amount of follicle stimulating hormone administered is in the range of approximately 2 to 20 iu.

More preferably the method of increasing ovulation to provide twinning further includes administering simultaneously with or after follicle stimulating hormone administration to the animal approximately 0.50 to 1.0 mg of a purified monoclonal antibody against follicle stimulating hormone formed by a hybridoma cell line.

In a preferred aspect of the present invention the method of inducing oestrus and/or increasing fecundity further includes administering to the animal to be treated an effective amount of a monoclonal antibody against follicle stimulating hormone (formed by a hybridoma cell line) simultaneously with or after follicle stimulating hormone administration.

The monoclonal antibody may be any of the antibodies described above. The monoclonal antibody may be administered in any suitable effective amount. For sheep and goats the monoclonal antibody may be administered in amounts from approximately 0.25 to 1.0 mg. For cattle the monoclonal antibody may be administered in amounts from approximately 1.0 to 3.0 mg. For pigs the monoclonal antibody may be administered in amounts from approximately 0.2 to 0.5 mg.

In the method of super ovulation, the monoclonal antibody may be administered for sheep and goats in amounts of from approximately 0.5 to 2.0 mg. For cattle the monoclonal antibody may be administered in amounts of from approximately 0.5 to 3.0

When administering FSH monoclonal antibodies for control of FSH in-vivo, an accurate assessment of the required dose is preferred.

The antibody dose suitable is dependent on several variables including:

    (i) the monoclonal antibody selected,
    (ii) FSH dose,
    (iii) time following FSH injection of antibody injection,
    (iv) species in which it is being used,
    (v) size of the animal, and
    (vi) number of times the animal has been previously treated.

The use of monoclonal antibodies for controlling FSH in vivo whether for use in superovulation, increasing fecundity i.e. twinning or for induction of oestrus as described for ewes and cows has similar application in goats, humans, dogs, cats, pigs and other mammals.

The amount of antibody necessary to be effective will vary with the particular antibody used, the species of animal, the level of FSH treatment and time of administration following FSH injection. For example, in a preferred form, where from approximately 15 to 20 iu of FSH is injected into a ewe, we have found that approximately 0.05 to 0.50 milligrams of antibody may be used depending on the variables already described. The monoclonal antibody treatment may be undertaken contemporaneously with the FSH treatment or may be undertaken after FSH treatment.

Monoclonal antibodies administered simultaneously or after FSH administration can modify the biological activity of FSH. This modification may reduce or enhance the activity of the FSH. Both enhancement and reduction of FSH bioactivity can be incorporated in the same regime. It is possible to achieve a 5 - 10 times increase in potency of FSH with "FSH-antibody" complex.

Preincubation, simultaneous injection of independent administration of FSH and the monoclonal antibody can enhance the activity of the FSH via the formation of antibody-FSH complexes, resulting in greater biological activity.

Equally so the formation of a different antibody-FSH complex can effectively reduce the bioactivity of the FSH or previously complexed FSH by nullifying the biological activity of the FSH.

In a still further aspect of the present invention there is provided a method for the detection of follicle stimulating hormone (FSH) in a mammal. The detection assay may be an immunofluorescent assay, radioimmuno-assay, enzyme-linked immuno assay or agglutination assay. For the IgM immunoglobulin class monoclonal antibodies an agglutination assay is preferred. A haemagglutination assay is preferred.

In a particularly preferred form the detection assay is a haemagglutination inhibition assay.

The sample to be tested may be of any suitable type. Sera, plasma, saliva or urine samples or extracts thereof may be used.

It would be understood that the presence of FSH may then be detected by visual means. Where FSH is present in the serum of interest, the FSH will bind to available binding sites on the monoclonal antibodies. When the FSH coated mammalian red blood cells are added there is effectively no antibody to bind to the antigen on the cells and there is a resultant inhibition of agglutination. Non-agglutinated cells subsequently slide around the round bottom of the solid support well to form a dark dot of cells.

The solid support may be of any suitable type. A microtitre plate or tray or test tube may be used. A microtitre plate or tray of the round bottom type is preferred. The monoclonal antibodies may be introduced as a solution. A phosphate buffered saline solution may be used.

In a further aspect the present invention provides a veterinary or pharmaceutical composition for the modification of the biological activity of FSH in mammals including an effective amount of a monoclonal antibody selected from those described above or mixtures thereof in a unit dosage form.

The veterinary or pharmaceutical composition may further include a veterinarily acceptable or pharmaceutically acceptable diluent carrier or excipient therefor. The monoclonal antibody may be provided in solution. A buffered saline solution may be used. A phosphate buffered solution may be used.

The present invention will now be more fully described with reference to the accompanying examples. It should be understood, however, that the description following is illustrative only and should not be taken in any way as a restriction on the generality of the invention described above.

## EXAMPLES

## MATERIALS AND METHODS

### Manufacture of Monoclonal Antibody to FSH

BALB/c mice were injected IP with 5 mg of a purified preparation of oFSH or pFSH (50 iu/mg, ammonium sulphate precipitated according to the method of Reichert 1975 followed by several ion exchange chromatography steps, in 50 ul of PBS emulsified in a ratio of 1:1 with Freund's Complete Adjuvant (FCA). This immunfication step was repeated four times at weekly intervals. One week after the final injection, mouse sera, obtained by tail bleeding were examined for FSH reactivity and specificity by radio-immunoassay (RIA). Mice having the highest serum titres were boosted four days prior to fusion with 100 ug of the same purified preparation of oFSH or pFSH (50 iu/mg) in 0.1 ml of PBS by tail-vein injection.

A primed mouse was killed by cervical dislocation and the spleen aseptically removed. Splenocytes were obtained by teasing of the spleen in 10ml GKN (0.8 g NaCl, 0.4 g KCl, 1.42 g $Na_2HPO_4$, $2H_1o$, 2.0 g

6

D-glucose and 0.01 g Phenol Rod in 1 litre of double distilled water). Solution: Cells were collected by centrifugation. NS-1 myeloma cells (2 x $10^7$) P3/NS1/1-Ag4-1, grown in tissue culture media (Dulbecco's Modified Eagles Medium, DME and sodium bicarbonate 0.2% w/v) were collected by centrifugation (200 g for 5 min. at room temperature) and resuspended in 10 ml GKN solution.

The splenocytes and NS-1 myeloma cells were mixed in a 50ml centrifuge tube (Falcon) and centrifuged at 70g for 5 minutes at room temperature. The supernatant was then poured off to leave a pellet of cells. These cells were resuspended to form a slurry, but gently tapping the tube, in a minimum of residual supernatant. 1.5ml of sterile 50% polyethyleneglycol at 37° C [PEG, Art. 9727 Polyethyleneglycol 4000, Merck] prepared by autoclaving 1.0g PEG with 1.0 ml of double distilled water in a sealed container, was then added to the slurry over 90 seconds, with gentle mixing. After a further 30 seconds of mixing, GKN at 37° C was carefully added over 2 minutes followed by a further 8 ml over 3 minutes. Finally 30ml GKN was then gently added down the side of the centrifuge tube.

The cell suspension was immediately centrifuged at room temperature for 3 minutes at 70g and the supernatant poured off. The tube was tapped gently to form a slurry of cells before resuspension in 100 ml of HAT selective media (tissue culture media containing 13.6 mg hypoxanthine, 0.19 mg aminopterin and 3.87 mg thymidine per litre of media). Samples of the cell suspension (0.5 ml) were then added to each well of eight 24-well flat bottom Linbro plates (Flow Laboratories) each containing 1.5 x $10^6$ rat thymocytes in 1.5ml HAT selective media. The thymocytes were prepared on the day prior to the fusion and incubated overnight at 37° C in a humidified 5% $CO_2$ atmosphere.

Thirteen days after fusion, supernatants were screened by RIA and Immunohistology. Selected wells were dilution cloned in 96-well flat bottom Linbro plates with thymocytes in HAT selective media.

Radioimmunoassay

ELISA (enzyme-linked immunosorbent assay) systems have often been used as a method of detecting monoclonal antibodies in hybridoma supernatants. However, in such a saystem antigen is presented in a solid phase, i.e. adherent to the plastic ELISA plate, and many scientists have reported false positive results (Miller, K.F., Goldsby, R.A., and Bolt, D.J. J. Endocr. (1987), 115: 283-288). To test for antibody which is required for use in liquid-phase applications, i.e. affinity chromatography and injection of antibody into animals as a suppressor of FSH activity, a liquid-phase assay was developed and is described below.

Radioimmunoassay Screening Procedure

Hybridomas secreting antibodies to ovine FSH were initially detected using a double-antibody RIA. A pure preparation of oFSH (75 u/mg from NIH, Bethesda, United States of America) is radioiodinated using Iodogen (Pierce, United States of America). In this procedure 5 ug of oFSH is incubated with 0.5 mg of $^{125}$I (Amersham IMS 30) and 50 ul of 0.1M Phosphate buffer, pH 7.4) in the presence of 1.2 mg of Iodogen coated onto a glass tube. After incubation for 10 minutes at room temperature the radioiodinated oFSH ($^{125}$I-oFSH) is separated from free $^{125}$I by chromatography on a 10 ml G-25 column.

Hybridoma supernatants (100 ul) are added to a polystyrene test tube (3DT tube, Johns) with 100 ul of 0.1% BSA/PBS containing approximately 100,000 counts per minute (cpm) of $^{125}$I = oFSH. After incubation for2 hours at room temperature 100 ul of a precipitating second antibody is added. The second antibody is an antimouse antibody made in rabbits (RAM, affinity purified Silenus Laboratories) conjugated to Sepharose 4B (CnBr- Sepharose 4B, Pharmacia). After vortexing tubes are left at room temperature for 1 hour and 1 ml of cold (4° C) phosphate buffer is added. Tubes are immediately spun at 3,000 rpm for 15 min. at 4° C. The non-antibody bound $^{125}$I-oFSH in the supernatant is aspirated and the pellet containing the $^{125}$I-oFSH-monoclonal antibody to oFSH - 2nd antibody complex is counted in a gamma spectro-photometer.

Immunohistology

Fresh tissues (pituitary glands) were collected from the local abattoir. The tissues were fixed in Bouins solution for two hours and then formalin overnight. After trimming, tissues were embedded in paraffin by processing through four 1 hour changes of absolute ethanol at 4° C , four changes of xylene at room temperature, and four changes of liquified paraffin at 56° C, and then left to solidify overnight at 4° C.

Tissue sections of 6 um were cut using a microtome (Leitz) and floated onto glass slides from a warm 1% gelatin (w/v) water bath. Slides were then incubated overnight at 37°C to adhere the sections. Prior to use, sections were rehydrated by 5 minute incubations in each of the following solutions; xylene, fresh xylene, absolute ethanol, fresh absolute ethanol, 70% ethanol and running water.

Neat hybridoma supernatants were placed directly onto the sections and the slides incubated in a humidified box for 2-3 hours at room temperature. After gentle rinsing with PBS, sections were covered with rabbit anti mouse IgG conjugated to horse radish peroxidase (RAM-HRP, Dakopatts, Denmark) diluted 1:75 in PBS containing 2% normal sheel serum and incubated for a further one hour. After rinsing the slides were submerged in a substrate solution containing 0.14 g 3,3'-diaminobenzamidine tetrachloride in 250ml 0.01M citrate buffer, pH 5, plus 250ul of 30% hydrogen peroxide. Within 10 minutes the reaction was stopped by rinsing the slides in distilled water.

Sections were counterstained with Harris' haematoxylin and lithium carbonate, and then dehydrated by one minute rinses through 70% alcohol, absolute alcohol, fresh absolute alcohol, xylene and fresh xylene for mounting. Supernatants were considered positive for FSH antibody if pituitary cell cytoplasm stained in the characteristic way described in the literature.

## Results

### Ovine FSH

The fusions performed on the spleen cells from mice immunized against oFSH yielded 2 hybridomas which produce antibodies to oFSH (see Table 1).

TABLE 1

|  | RIA | Histology |
| --- | --- | --- |
| BFR87-70 | 30% | + |
| BFR87-124 | 24% | + |
| a. max. binding of total $^{125}$I-oFSH added | | |
| b. + is staining of cytoplasm of pituitary cells | | |

### Improvement of Biological Activity

An improvement of the biological activity of FSH preparations can be achieved by affinity chromatography using monoclonal antibodies to FSH. There is also evidence of an increased potency of injected FSH when the FSH is coupled to the monoclonal antibodies.

The extraction of purified, biologically active FSH from crude extracts by affinity chromatography confirms the specificity of the antibody and allows accurate assessment of the biological properties of FSH.

### Affinity Chromatography

Approximately 200mg of each monoclonal antibody is purified (from cell culture or ascites fluid) and coupled to Affigel 10 at 16mg/ml of gel. The columns are prepared for each affinity gel preparation, with a small portion (200 ul) of gel for use in establishing optimal binding and eluting conditions. Optimal conditions were investigated by incubating 10 ul of gel in 200 ul of buffers at various pH values. After 1 hour at room temperature, the test tubes are centrifuged and any non-bound $^{125}$I-oFSH agitated. The gel containing any $^{125}$I-oFSH is then counted in a gamma spectrophotometer.

| OPTIMAL BINDING FOR BFR87-70 | | | |
|---|---|---|---|
| Buffer | | Counts Bound | % Counts Added |
| 0.1 Ethanolamine-HCl | pH 11.0 | 2,610 | 2 |
| PBS | pH 7.4 | 68,920 | 47 |
| 0.1M Phosphate | pH 7.0 | 64,490 | 44 |
| 0.1M Citrate/Citric Acid | pH 6.0 | 58,710 | 40 |
| 0.1M Citrate/Citric Acid | pH 5.5 | 54,240 | 37 |
| 0.1M Citrate/Citric Acid | pH 5.0 | 53,270 | 36 |
| 0.1M Citrate/Citric Acid | pH 4.5 | 38,460 | 26 |
| 0.1M Citrate/Citric Acid | pH 4.0 | 19.550 | 13 |
| 0.1M Citrate/Citric Acid | pH 3.5 | 3,820 | 2.6 |
| 0.1M Citrate/Citric Acid | pH 3.0 | 3,490 | 2.4 |

For optimal elution, a series of tubes were incubated with 10 ul gel plus $^{125}$I-oFSH diluted in PBS. These tubes withbound $^{125}$I-oFSH, were further incubated with buffers at various pH values, spun and counted.

| OPTIMAL ELUTION FOR BFR87-70 | | | |
|---|---|---|---|
| Buffer | | Counts Bound | % Counts Eluted |
| 0.1 Ethanolamine-HCl | pH 11.0 | 8,270 | 91 |
| PBS | pH 7.4 | 90,780 | 0 |
| 0.1M Phosphate | pH 7.0 | 86,560 | 6 |
| 0.1M Citrate/Citric Acid | pH 6.0 | 85,180 | 8 |
| 0.1M Citrate/Citric Acid | pH 5.5 | 80,120 | 8 |
| 0.1M Citrate/Citric Acid | pH 5.0 | 77,010 | 21 |
| 0.1M Citrate/Citric Acid | pH 4.5 | 35,800 | 46 |
| 0.1M Citrate/Citric Acid | pH 4.0 | 27,380 | 67 |
| 0.1M Citrate/Citric Acid | pH 3.5 | 12,160 | 87 |
| 0.1M Citrate/Citric Acid | pH 3.0 | 9,160 | 91 |
| * Relative to counts bound in first incubation with PBS. | | | |

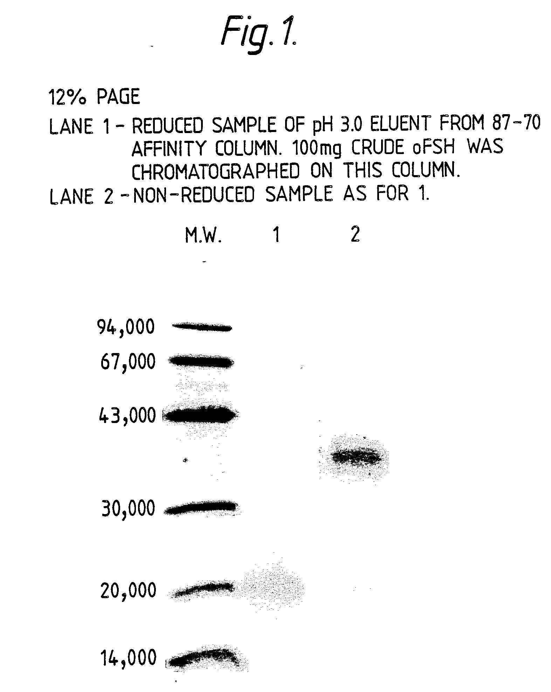

Optimal binding occurs using PBS as the binding buffer while maximum elution from the gel matrix, of $^{125}$I-oFSH, is achieved with 0.1M citrate/citric acid pH 3.0 buffer. This is preferred to the pH 11.0 buffer as FSH is less susceptible to damage at pH 3.0. This is illustrated in Figure 1.

This protocol was tested in a 2ml column, which indicated that this procedure was suitable (Table 2).

## Affinity Column (2 ml)

| | | |
|---|---|---|
| $^{125}$I-oFSH added | 3,047,800 | |
| Counts washed through | 840,000 | (28%) |
| Counts bound | 2,200,000 | 72% |
| Counts eluted with pH 3.0 citrate/citric acid | 2,116,550 | 96% of bound counts |

Large scale trials can be undertaken by either column or bath methods. The affinity-purified FSH can be tested for biological activity using the hCG augmentation assay for FSH of Steelman and Pohley (Endocrinology, 53:604-616, 1953) or the porcine testes receptor assay of Maughium-Rogister et al, (Eur. J. Biochem. 86:121-131, 1978).

This purified preparation of FSH will allow an exact dose of FSH to be given. Batch variations found in crude preparations and the problems caused will be alleviated.

These antibodies to ovine and porcine FSH will also allow the investigation of enhancement of FSH activity by the coupling of biological FSH and its antibody and injecting this complex into the object. This treatment may decrease the dosage rate of FSH substantially and hence decrease the cost of FSH treatment since the major cost of the treatment is the purified FSH.

Finally, it is to be understood that various other modifications and/or alterations may be made without departing from the spirit of the present invention as outlined herein.

## Claims

1. A process for producing a monoclonal antibody against follicle stimulating hormone (FSH) which process includes
providing a B cell capable or producing antibodies against FSH, and a myeloma cell;
fusing the B cell with the myeloma cell;
propagating a hybridoma formed thereby; and
harvesting an antibody produced by said hybrodima.

2. A process according to claim 1 wherein the B cell capable of producing antibodies is obtained from an animal immunised with ovine follicle stimulating hormone (oFSH) or an immunogenic fragment thereof; and
the myeloma cell line is an NS-I type myeloma cell line.

3. A process according to claim 2 wherein the fusion step includes mixing somatic cells with myeloma cells in the presence of a fusion promoting agent selected from Sendai virus and polyethylene glycol.

4. A process according to claim 3 wherein the hybridoma propagation step includes
detecting antibody-producing hybridomas utilising a radioimmunoassay together with immunohistology;
subsequently cloning the fused cell hybrid selected into individual antibody-producing cell lines; and
propagating each cell line in vivo or in vitro.

5. A continuous cell line which produces a monoclonal antibody against follicle stimulating hormone including a hybridoma formed by fusing a B cell capable of producing antibodies against follicle stimulating hormone with a myeloma cell.

6. A continuous cell line according to claim 5 formed by fusing an NS-I type myeloma cell with a spleen B cell obtained from a BALB/c mouse immunised with ovine follicle stimulating hormone.

7. A continuous cell line selected from BFR87-70 and BFR87-124 as hereinbefore described.

8. A monoclonal antibody against follicle stimulating hormone produced from a continuous cell line which produces a monoclonal antibody against follicle stimulating hormone including a hybridoma formed by fusing a B cell capable of producing antibodies against follicle stimulating hormone with a myeloma cell.

9. A monoclonal antibody produced from a continuous cell line selected from BFR87-70 and BFR87-124 as hereinbefore described.

10. A method of purifying follicle stimulating hormone (FSH) which method includes
providing
a source of impure FSH, and
a monoclonal antibody against FSH bound to a matrix;
passing the impure FSH through the monoclonal antibody matrix to capture FSH; and
eluting the captured FSH from the monoclonal antibody matrix.

11. A method according to claim 10 wherein the follicle stimulating hormone is an ovine follicle stimulating hormone (oFSH); and
the monoclonal antibody bound to the matrix is selected from BFR87-70 and BFR87-124 as hereinbefore described.

12. A method according to claim 11 wherein the matrix is a chromatographic gel and the elution step includes stepwise elutions with an 0.1 molar citrate/citric acid solution.

13. A purified follicle stimulating hormone when prepared by a method according to claim 10.

14. A method of inducing oestrus and/or increasing fecundity in female animals, which method includes administering to the animal an effective amount of a purified follicle stimulating hormone according to claim 13.

15. A method according to claim 14 wherein the animal to be treated is a sheep or goat and the amount of purified FSH administered is in the range of from approximately 2 to 10 iu.

16. A method according to claim 15 further including administering simultaneously with, or after, FSH administration to the animal approximately 0.25 to 1.0 mg of an purified monoclonal antibody against FSH formed by a hybridoma cell line.

17. A method according to claim 14 wherein the animal to be treated is a cow and the amount of FSH administered is in the range of approximately 2 to 20 iu.

18. A method according to claim 17 further including administering simultaneously with or after FSH administration to the animal approximately 1.0 to 3.0 mg of a purified monoclonal antibody against FSH formed by a hybridoma cell line.

19. A method of substantially increasing the number of ova produced during a single oestrus cycle in a female animal, which method includes administering to the animal an effective amount of a purified FSH according to claim 13.

20. A method according to claim 19 wherein the animal to be treated is a sheep or goat and the amount of FSH administered is in the range of approximately 4. to 20 iu.

21. A method according to claim 20 further including administering simultaneously with, or after, FSH administration to the animal approximately 0.5 to 2.0 mg of a purified monoclonal antibody against FSH formed by a hybridoma cell line.

22. A method according to claim 19 wherein the animal is a cow and the amount of FSH administered is in the range of 4 to 20 iu.

23. A method according to claim 22 further including administering simultaneously with or after FSH administration to the animal approximately 0.5 to 3.0 mg of a purified monoclonal antibody against FSH formed by a hybridoma cell line.

24. A method of increasing ovulation to provide twinning in female animals, which method includes administering to the animal an effective amount of a purified FSH according to claim 13.

25. A method according to claim 24 wherein the animal is a sheep, goat or cow and the amount of FSH administered is in the range of approximately 2 to 20 iu.

26. A method according to claim 24 further including administering simultaneously with or after FSH administration to the animal approximately 0.05 to 1.0 mg of a purified monoclonal antibody against FSH formed by a hybridoma cell line.

27. A veterinary or pharmaceutical composition including
an effective amount of a monoclonal antibody against follicle stimulating hormone (FSH) formed by a hybridoma cell line; and
a veterinarily or pharmaceutically acceptable diluent excipient or carrier therefor.

28. A pharmaceutical or veterinary composition according to claim 27 further including an effective amount of a purified FSH according to claim 13.

29. A veterinary or pharmaceutical composition according to claim 28 wherein the purified follicle stimulating hormone is a purified ovine follicle stimulating hormone.

30. A veterinary or pharmaceutical composition according to claim 29 wherein the composition is a buffered saline solution.

# Fig.1.

12% PAGE

LANE 1 – REDUCED SAMPLE OF pH 3.0 ELUENT FROM 87–70
AFFINITY COLUMN. 100mg CRUDE oFSH WAS
CHROMATOGRAPHED ON THIS COLUMN.
LANE 2 – NON–REDUCED SAMPLE AS FOR 1.